# EUROPEAN PATENT SPECIFICATION

(11) **EP 4 061 286 B1**
(45) Date of publication and mention of the grant of the patent: **19.07.2023**
(21) Application number: 20804308.3
(22) Date of filing: 17.11.2020
(51) Int. Cl.: A61F 2/40

(54) **GLENOID COMPONENT FOR SHOULDER PROSTHESIS AND RELATED SHOULDER PROSTHESIS**
GLENOIDKOMPONENTE FÜR SCHULTERPROTHESE UND ZUGEHÖRIGE SCHULTERPROTHESE
COMPOSANT GLÉNOÏDE POUR PROTHÈSE D'ÉPAULE ET PROTHÈSE D'ÉPAULE ASSOCIÉE

(30) Priority: 18.11.2019 IT 201900021432
(43) Date of publication of application: 28.09.2022
(73) Proprietor: Limacorporate S.p.A., 33038 San Daniele Del Friuli (UD) (IT)
(72) Inventor: POON, Peter Channel, 0620 Auckland (NZ); IZQUIERDO, Rolando, Crystal Lake, IL 60014 (US); FATTORI, Andrea, 33038 San Daniele del Friuli (UD) (IT); PRESSACCO, Michele, 33038 San Daniele del Friuli (UD) (IT); DEL NEGRO, Nicola, 33038 San Daniele del Friuli (UD) (IT); BORMANN, Kurt, Columbia, Missouri 65203 (US)
(74) Representative: Botti & Ferrari S.p.A.
(86) International application number: PCT/EP2020/082348
(87) International publication number: WO 2021/099296

(56) References cited:
- EP-A1- 1 776 936
- US-A1- 2005 049 709
- US-A1- 2010 087 927
- US-A1- 2010 125 336
- US-A1- 2012 209 392
- US-A1- 2012 239 155
- US-A1- 2013 282 129
- US-A1- 2017 172 764
- US-A1- 2017 202 674

## Description

### Technical field

The present invention relates to a glenoid component for shoulder prosthesis comprising a glenoid coupling and a prosthetic coupling.

The present invention also relates to a shoulder prosthesis comprising a glenoid component.

In general, the present invention finds application in the prosthetic orthopedic field for shoulder surgery.

### Prior art

Within the present technical field, the erosion of the glenoid surface is a fairly frequent factor and must be taken into account in the context of shoulder prosthetic surgery.

Indeed, for a good result in the medium-long term it is necessary, during surgery, not only to re-establish the right tension of the soft tissues, but also to try to recover any bone deficits that may be present and cause sub-dislocation problems of the humeral head.

There are various types of glenoid erosions. Although the posterior ones are the most frequent, there are upper erosions, deformities of the glenoid with lower inclinations and central defects. All of these individual defects are often combined together in a specific case. For instance, the lower deformation of the glenoid combined with the rear erosion is one of the fairly frequent conditions in osteo-arthritic patients.

It is therefore important, intra-operatively, to identify the right type of defect and correct it by returning the version of the glenoid to a neutral condition. For instance, a prosthetic humeral head that articulates off-center could cause excessive and premature wear of the polyethylene component and at the same time cause failure of the glenoid component due to the phenomenon called "rocking-horse".

In the prior art, this type of criticality has sometimes been remedied by means of an asymmetrical milling of the glenoid surface in order to bring back the glenoid to an original neutral version.

Although this expedient constitutes a still adopted solution, it has drawbacks, in particular linked to the fact that the asymmetrical milling of the surface has the disadvantage of removing a significant portion of bone to correct the defect, thus making it necessary to subsequently lateralize the components to properly tension the soft tissues.

A further methodology used nowadays involves the insertion of a bone graft into the area with a deficit. A disadvantage of this technique is the difficulty in retrieving the graft. In fact, the graft is usually taken from a bone bank or directly from the patient with operations that can be painful and have long recovery times (e.g. bone graft removal from the iliac crest). Furthermore, this methodology has the disadvantage of having an implant technique that is particularly complex and exposed to a possible human error, which could lead to a non-optimal result in the bone deformity recovery and a consequent non-optimal result in the prosthesis connection.

The use of an increased prosthetic component, such as the one covered by the present invention, allows overcoming these problems. In particular, the implant technique is much simpler for any user with ordinary knowledge of the field and at the same time allows obtaining good results.

Document US 2012/209392 A1 relates to a glenoid assembly including a glenoid plate configured for fixation to a glenoid bone for a reverse shoulder prosthesis; a glenosphere configured for connection to the glenoid plate; and an adjustment plate, wherein the adjustment plate has a connection for directly engaging the glenosphere, and wherein the adjustment plate has an articulation for directly engaging the glenoid plate at a variable angular orientation.

Document US 2010/087927 A1 relates to a glenoid component used to resurface the scapula. The glenoid component in a reverse shoulder is convex rather than concave.

Document US 2010/125336 A1 relates to a reverse shoulder prosthesis comprising a part spherical, convex, surface which is less than a hemisphere. The part spherical surface does not have a rotational axis of symmetry.

Document US 2012/239155 A1 relates to a glenoid component of a shoulder prosthesis, including a spherical articulating surface and an opposite bone contacting surface.

Document US 2005/049709 A1 relates to glenoid component comprising a metal body of which the inner face is adapted to be immobilized on the glenoid cavity of a shoulder and of which the outer face bears a concave articulating surface adapted to cooperate with a humeral component. This articulating surface extends on the periphery by a convex surface forming, at least in part, the edge of the body.

### Summary

An object of the present invention is to overcome drawbacks of the prior art.

A further object is to provide a component that is adapted to confer a particular effectiveness to the glenoid prosthesis system.

A further object is to provide a component that is adapted to confer greater convenience of application or of use by a surgeon.

A particular object of the present invention is to provide a device that allows recovering the typical bone deficits with different severities.

A further particular object of the present invention is to provide a solution that allows an immediate and standardized selection in the operating phase by a surgeon.

Still a further particular object is to provide a solution that allows a simple application by a surgeon.

Another particular object is to provide a structure of increased specific dimensions for the correction of bone deficits.

A further particular object is to provide a component that allows a quick coupling with further devices for prosthetic surgery such as bone pins.

Finally, a particular object of the invention is to provide a device that is particularly suitable for the standard productive process of prosthetic components.

These and other objects are achieved by means of the glenoid component according to the features of claim 1.

A general idea underlying the present invention is to provide a glenoid component for shoulder prosthesis, comprising a glenoid coupling surface adapted to be fixed to a bone and comprising a first convex portion having a first outer edge that defines a development plane, and a prosthetic coupling surface, opposite the glenoid coupling surface and adapted to house a second prosthetic component of the shoulder prosthesis, the prosthetic coupling surface comprising a second portion having a second outer edge that defines a reference plane. The glenoid component provides a predetermined offset between the development plane and the reference plane so as to define an increased thickness of the glenoid component, said increased thickness being adapted to properly compensate for a bone deficit.

Advantageously, the present invention provides the surgeon with a standard solution for a range of typical deformity situations with different severity found in the shoulder surgery.

In particular, the increased thickness of the glenoid component is defined by a maximum offset provided by a respective predetermined offset between the development plane defined by the first outer edge and the reference plane defined by the second outer edge. Preferably, the increased thickness of the glenoid component is defined by a maximum offset equal or larger than 15%, more preferably equal or larger than 25%, of the diameter of the glenoid component.

Preferably, the predetermined offset is configured to vary a longitudinal distance along the perimeters of the first outer edge and of the second outer edge, thus defining an angle between the development plane and the reference plane.

Advantageously, the present solution allows re-establishing the correct conformation in presence of bone deficits of the angular type, in particular at the posterior spherical glenoid surface.

Still preferably, the angle between the development plane and the reference plane is comprised between 2° and 30°, preferably comprised between 5° and 25°, more preferably available with increases of 5°.

Advantageously the present ranges are particularly effective in correcting typical bone deficits of the angular type and do not involve mechanical resistance problems of the component and do not require further milling operations.

According to an alternative example, the predetermined offset is configured to substantially maintain a longitudinal distance along the parameters of the first outer edge and the second outer edge constant, wherein the development plane and the reference plane are substantially parallel to each other in defining the standard increased thickness.

Advantageously, this alternative example is particularly suitable for central erosions of the glenoid, allowing the original thickness recovery.

Preferably, the longitudinal distance is comprised between 2 and 10 mm, more preferably comprised between 4 and 6 mm.

Advantageously, the present ranges are particularly suitable for the typical bone deficits of the central type in which it is further necessary to correctly tension the soft tissues.

The glenoid component further comprises a substantially truncated cone-shaped glenoid coupling element, protruding from said glenoid coupling surface and adapted to be coupled to a bone pin, the glenoid coupling element developing along an axis which is substantially perpendicular to the reference plane.

Advantageously, the present solution is suitable for being associated with bone pins of different lengths, making the glenoid component according to the present invention suitable for a plurality of different solutions.

Still preferably, the first convex portion of the glenoid component provides a substantially constant curvature radius, preferably comprised between 25 and 35 mm, more preferably between 27 and 30 mm, whereas the second portion is preferably concave.

Advantageously such conformation allows replicating in the best possible way the bone curvature radius of the original glenoid and to obtain the correct seat for the glenoid component using the same tool for all of the conformations.

The first outer edge of the glenoid coupling surface is substantially circular or oval or glenoid-shaped, whereas the second outer edge of the prosthetic coupling surface is substantially circular or oval.

Advantageously, a circular conformation allows an orientation of the glenoid component in a continuous way, which covers all types of defects over 360°.

The oval or glenoid-shaped conformations represent the optimal condition for a prosthesis of the anatomical type, in which the humeral head articulates on the glenoid surface, as it happens, precisely, anatomically. Indeed, these conformations are particularly suitable when the surgeon deems it necessary to use an anatomical prosthesis, for example in the case of a young patient with integrity of the rotator cuff.

Still preferably, the glenoid component further comprises at least two holes, preferably four holes, adapted to house fixing screws of the component.

Advantageously, the correct choice of the number of fixing screws allows a correct orientation of the component even in the case of hybrid bone deficits.

Preferably, according to an alternative embodiment, said holes are adapted to house respective angular locking screws.

Still preferably, the predetermined offset comprises a first offset at said holes and a second offset at a glenoid coupling element.

Said first and second offsets, in a particularly advantageous embodiment, have the purpose of providing an additional thickness in the screw holes, in order to introduce an angularly stable locking element.

Preferably, the first offset comprises a first threaded portion, adapted to house a peripherally threaded closure element, and preferably the second offset comprises a second threaded portion, adapted to house a head of one of the angular locking screws.

Advantageously, said solution allows introducing the angular locking screws in a first step, leaving the surgeon the possibility to orient them in the most appropriate direction and, in a second step, to lock the inserted screw by inserting the closure element, regardless of the direction chosen for the angular locking screw in the first step.

Preferably, the glenoid coupling surface comprises a trabecular porous structure, adapted to increase the stability and osteo-integration of said glenoid component.

Still preferably, the glenoid component further comprises retentive elements protruding from the prosthetic coupling surface, preferably said retentive elements being adapted to house and retain an anatomic liner, preferably of polyethylene, which, advantageously, replicates the surface on which the humeral head articulates.

Preferably, the present invention provides for a set including a plurality of glenoid components, having respective different predetermined offsets so as to define different standard increased thicknesses, adapted to compensate for different bone deficits. Advantageously, these glenoid components provide different standard increased thicknesses, which are in particular available to the surgeon in a plurality of discrete increments (i.e. increasing of 5%, or of 7.5%, or of 10%, or yet different increments) for properly compensating the specific bone deficit encountered in a patient.

According to a further aspect of the invention, a shoulder prosthesis comprising at least one second prosthetic component and a glenoid component made according to what has been described above is provided.

Further features and advantages will become more apparent from the following detailed description of preferred, non-limiting, embodiments and examples of the present invention, and from the dependent claims which outline preferred and particularly advantageous embodiments of the invention.

### Brief description of the drawings

The invention is illustrated with reference to the following figures, given by way of non-limiting example, in which:
- Figures 1A and 1B illustrate front and rear perspective views of an embodiment of a glenoid component according to the present invention;
- Figure 2 illustrates a possible coupling of the glenoid component of Figure 1 with a bone pin;
- Figures 3A and 3B illustrate an exemplifying coupling of the glenoid component of Figures 1A and 1B on a scapula;
- Figure 4 illustrates a side view of the glenoid component of Figures 1A and 1B;
- Figure 5 illustrates possible variants of the glenoid component of Figures 1A and 1B;
- Figure 6 illustrates a further variant of a glenoid component of Figures 1A and 1B;
- Figure 7 illustrates the glenoid component of Figures 1A and 1B coupled with fixing screws;
- Figure 8 illustrates an example of a glenoid component;
- Figure 9 illustrates a side view of the glenoid component of Figure 8;
- Figure 10 illustrates an exploded view of an exemplifying shape of the glenoid part of a reverse shoulder prosthesis comprising the glenoid component of Figures 1A and 1B;
- Figures 11A and 11B illustrate front and rear perspective views of an example of a glenoid component;
- Figures 12A and 12B illustrate front and rear perspective views of a variant of the glenoid component of Figures 11A and 11B;
- Figure 13 illustrates possible variants of the glenoid component of Figures 11A and 11B;
- Figure 14 illustrates a possible coupling with an anatomic liner of the glenoid component of Figures 11A and 11B;
- Figure 15 illustrates an exploded view of a further exemplifying shape of the glenoid part of a reverse shoulder prosthesis comprising the glenoid component of Figures 11A and 11B;
- Figures 16A and 16B illustrate a further exemplifying coupling of the glenoid component of Figures 11A and 11B on a scapula;
- Figure 17 illustrates a perspective view of a further embodiment of a glenoid component according to the present invention;
- Figure 18 illustrates a further perspective view of the variant of Figure 17 coupled with a fixing screw;
- Figure 19A and 19B illustrate a perspective view and a sectional view of the variant of Figure 17 coupled with a fixing screw.

In the different figures, analogous elements will be identified by analogous reference numbers.

### Detailed description

With reference to the enclosed Figures, reference number 100 globally indicates an embodiment of a glenoid component for a shoulder prosthesis made according to the present invention.

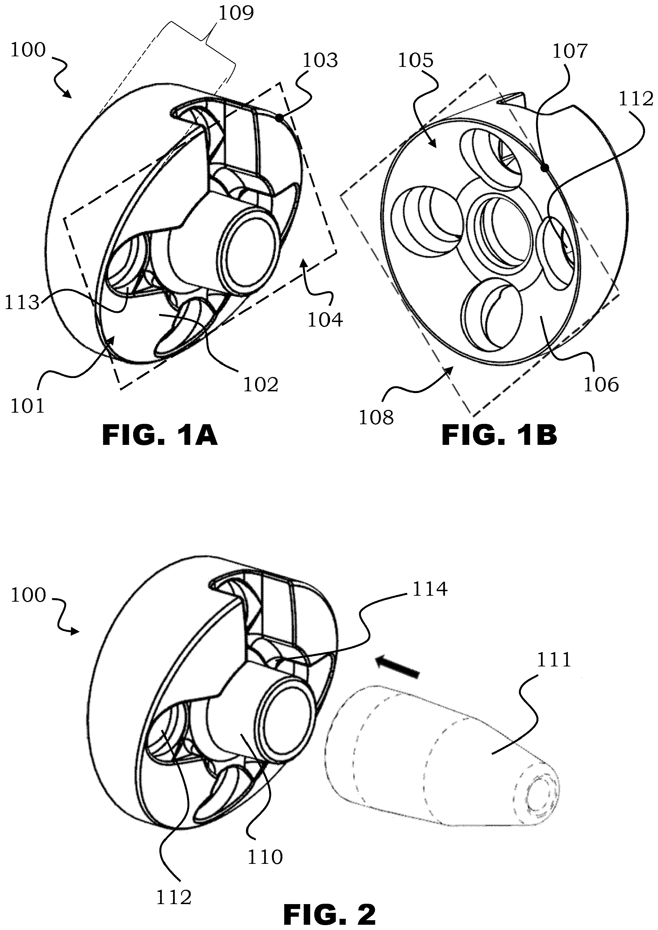

Figure 1 represents an embodiment of the glenoid component 100 comprising a glenoid coupling surface 101, adapted to be fixed to a bone. The glenoid coupling surface 101 comprises a first convex portion 102 having a first outer edge 103 which defines a development plane 104. The glenoid component 100 further comprises a prosthetic coupling surface 105, opposite the glenoid coupling surface 101 and adapted to house a second prosthetic component of the shoulder prosthesis. The second prosthetic component is preferably a humeral component of the shoulder prosthesis.

The prosthetic coupling surface 105 comprises a second portion 106 having a second outer edge 107 which defines a reference plane 108. The second portion 106 is preferably concave.

The glenoid component provides a predetermined offset 109 between the development plane 104 and the reference plane 108.

Furthermore, the glenoid component 100 comprises a substantially truncated cone-shaped glenoid coupling element 110, protruding from the glenoid coupling surface 101. The glenoid coupling element 110 may be made integral or removable with respect to the body of the glenoid component 100.

As visible in Figure 2 the glenoid coupling element 110 is adapted to be coupled to a bone pin 111. Since this coupling is of the modular type, preferably a conical coupling of the morse cone type, there is the possibility of using bone pins 111 of various length, based on the bone quality and on the stability desired by the surgeon during the operation. The freedom of use with the same glenoid component 100 is consequently maximum for a surgeon. The glenoid coupling element 110 develops along an axis that is substantially perpendicular to the reference plane 108.

In the present embodiment the glenoid component 100 comprises four through-holes 112, adapted to house fixing screws of the glenoid component 100. A recess 113 with a preferably substantially parallelepiped section at the glenoid coupling surface 101 corresponds to each hole 112. Furthermore, preferably around the glenoid coupling element 110 a circular recess 114 in connection with the recesses 113 is provided.

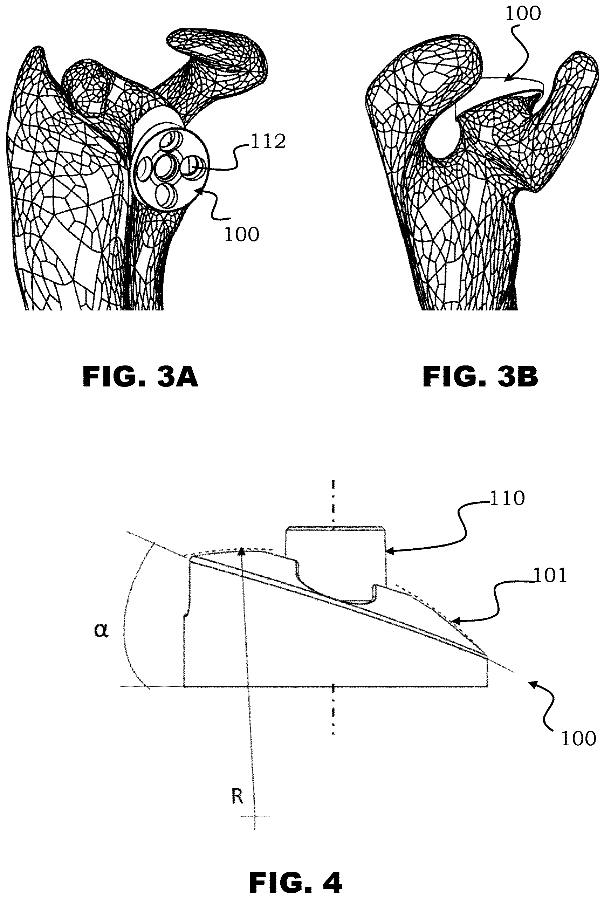

In Figures 3A and 3B a possible exemplifying application of the glenoid component 100 on a scapula is visible. In the present exemplifying and non-limiting embodiment both the first outer edge 103 of the glenoid coupling surface 101 and the second outer edge 107 of the prosthetic coupling surface 105 are substantially circular. The present configuration allows an orientation of the glenoid component 100 in a continuous way, which covers all types of defects over 360°. Nothing prevents the use of different conformations. For instance, it is possible to provide a first outer edge 103 of the glenoid-shaped glenoid coupling surface 101, which represents the optimal condition for a prosthesis of the anatomic type, wherein the humeral head articulates on the glenoid surface.

However, this exemplifying solution is only suitable for reverse prosthesis. Reverse prosthesis means a prosthesis that does not rely on the action of the rotator cuff muscles but relies on the deltoid muscle. In other words, it is a prosthesis with the head on the scapular glenoid that articulates on a spherical liner positioned on the humeral part, using the lever arm of the deltoid. This type of prostheses are used in patients with irreparable injuries of the rotator cuff with loss of function and strength of the shoulder, precisely, in which it is not possible to adopt the rotator cuff muscles to make the shoulder prosthesis work.

Figure 4 represents a side view of the glenoid component 100 in which the first convex portion 102 is particularly visible, said first portion 102 preferably providing a curvature radius R that is substantially constant, preferably comprised between 25 and 35 mm, more preferably between 27 and 30 mm. Said conformation allows replicating the bone curvature radius of the original glenoid in the best possible way and obtaining a correct seat for the glenoid component 100 using the same tool for all of the configurations.

Furthermore, the embodiment represented in Figure 4 has a predetermined offset 109 configured to vary a longitudinal distance along the perimeters of the first outer edge 103 and of the second outer edge 107, thus defining an angle α between the development plane 104 and the reference plane 108.

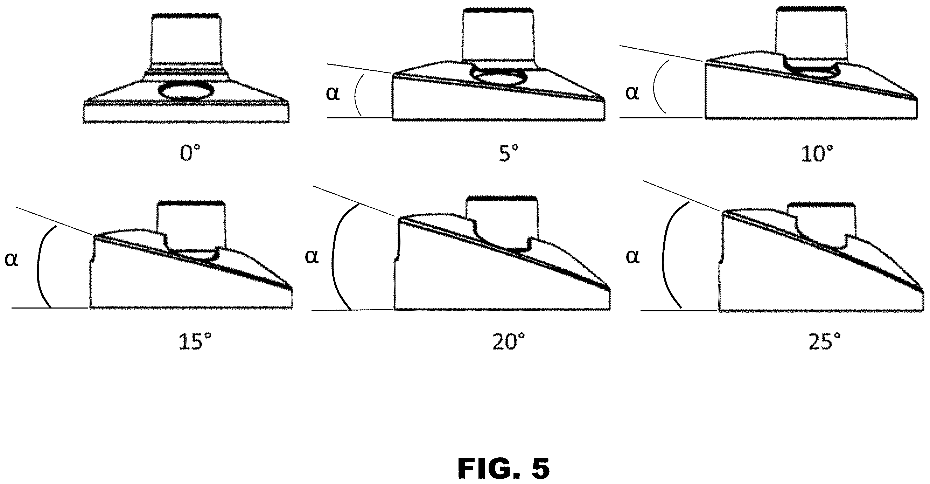

Figure 5 represents different variants of said embodiment. Considering that in all cases it is necessary to establish the right correction, the glenoid component 100 according to the different variants provides different inclinations of the development plane 104 of the first convex portion 102 and thus from different angles α, to be considered based on the severity of the defect. It should be borne in mind that even an excessive correction with respect to the neutral position (in which a patient is in an upright position with the limb under examination adducted along the side and with the palm of his hand facing the lateral side of the corresponding lower limb) may also cause problems in the medium-long term to the patient.

As stated, the first portion 102 of the glenoid coupling surface 101 is convex with a curvature radius R that is constant for all of the configurations to use the same tool, which in the present case is represented by a cutter with the same curvature radius. It is therefore sufficient to orient the cutter in the correct way based on the defect. In particular, Figure 5 represents variants with angles 0° to 25° with increases by 5°. Clearly, said representation is only illustrative and non-limiting of the scope of protection defined by the appended claims. This example is selected since the angles represented are the most frequent and an increase by 5° between one variant and the other is considered the minimum appreciable. As stated, however, it is possible to make the glenoid component 100 with all possible intermediate angles, based on the surgeon's specific needs, such as 17,5° or 12° angles. For glenoid components 100 with greater angulations longer bone pins 111 are used, as the added material part covers a part of the exposed surface of the pin.

Preferably, the glenoid component 100 is not made with angles greater than 30°, preferably not greater than 25°, to avoid mechanical strength complications of the glenoid component 100 increasing the lever arm between point of application of force and bone. Generally, to correct very severe deficits, such as with an angle of 35°, it is preferable to use a glenoid component 100 combined with a preliminary milling operation on an inclined axis, with respect to the eroded surface. However, nothing prevents the provision of a more resistant glenoid component 100 and adopting higher angles of the development plane 104.

Moreover, the increased thickness of the glenoid components shown in Fig. 5 are defined by a maximum offset between the glenoid coupling surface and the prosthetic coupling surface that ranges from about: 10% for 0°, 20% for 5°, 25% for 10°, 33% for 25°, 43% for 20°, 50% for 25° with respect to the respective diameter of the glenoid component.

The position of the convexity curvature center of the glenoid component 100 is selected, in the various configurations, in order to ensure the desired angle α and at the same time to ensure a minimum thickness of the glenoid component 100, still in order to avoid any mechanical seal problems.

In the present case with a first circular outer edge 103 and second circular outer edge 107 it is sufficient to position the thickest part of the glenoid component 100 along the direction of the bone deficit.

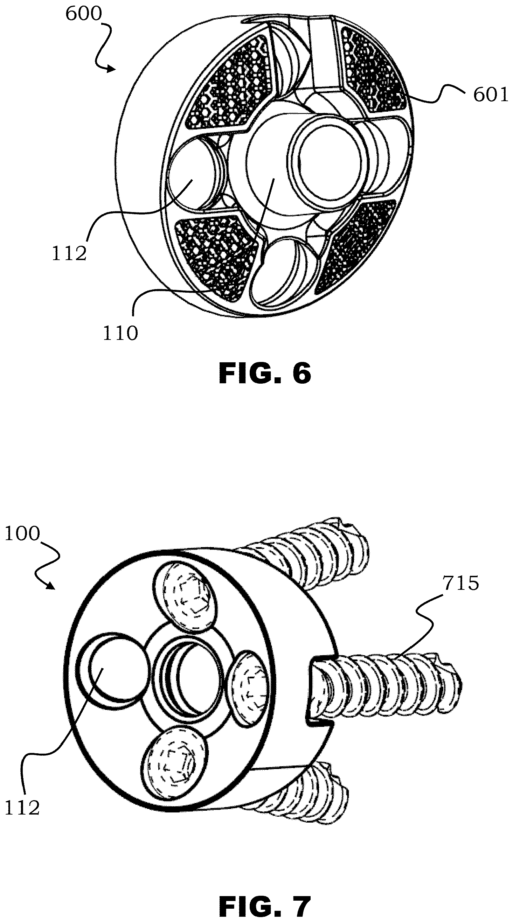

Figure 6 further represents a variant 600 of the glenoid component 100 in which a porous/trabecular structure 601 is provided on the glenoid coupling surface 101, in order to increase the stability and osteo-integration of the glenoid component 100 at the bone. A trabecular structure is a structure that incorporates that characteristic of bone tissue, with lamellae arranged forming intercommunicating cavities.

As stated and as most visible in Figure 7, the glenoid component 100, in the present embodiment, has four holes 112 adapted to house fixing screws 715 to the bone surface. In said embodiment the four holes 112 allow choosing the position and the optimal number of fixing screws 715 for the fixing even in the case of glenoid component 100 oriented along hybrid defects, for instance an inferior-posterior deficit inclined by 45°.

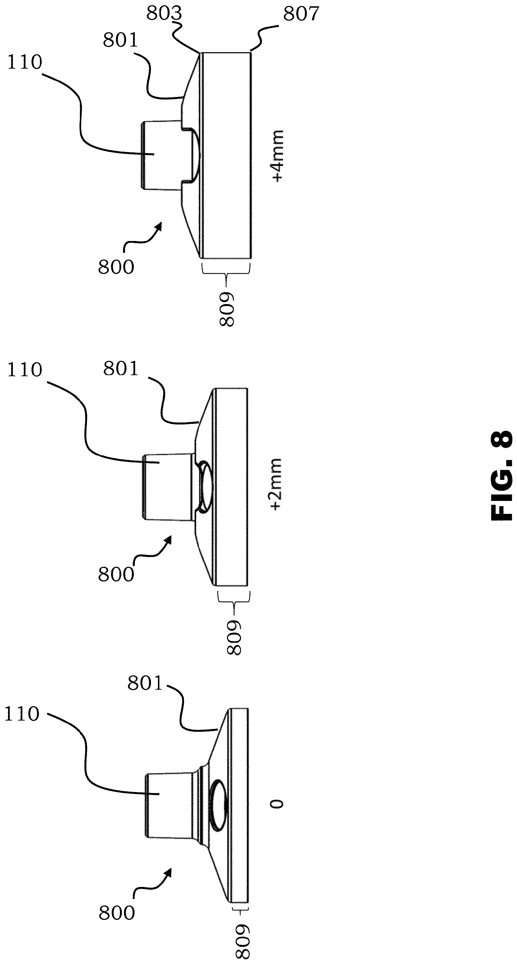

Figure 8 represents a different example 800 in different variants. In said example the predetermined offset 809 is configured to maintain a longitudinal distance substantially constant along the perimeters of the first outer edge 803 and of the second outer edge 807, wherein said development plane 104 and said reference plane 108 are substantially parallel to each other. Said example is particularly suitable in the case of central erosions of the glenoid. Indeed, under this condition, after an on-axis milling operation that eliminates the deficit part, a glenoid component 800 is required, which is increased not angularly but in terms of flat thickness, which brings the milled articular surface back to the same original height. The variants represented in Figure 8 differ from each other in the different thicknesses S.

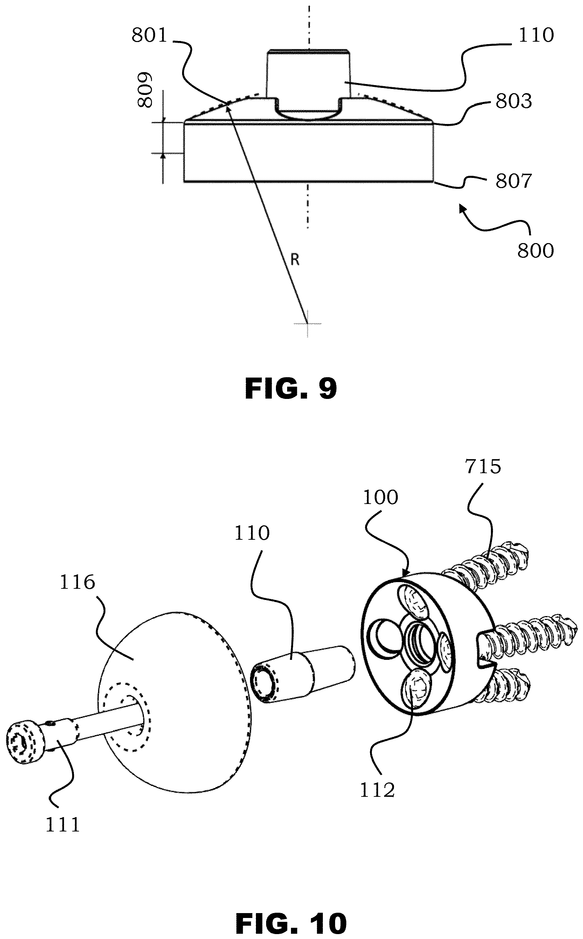

The glenoid coupling surface 801, as visible in Figure 9, has in this case as well a first convex portion 802, with a constant curvature radius R. The convexity curvature center is, in this case, always in axis with the glenoid component 800. Therefore, in the preparatory phase, the seat for the glenoid component 800 is made with a cutter with the same radius R, seat which is positioned coaxially and actuated until the central defect is eliminated. Additionally to its use to compensate for central bone deficits, the present example is suitable for correctly tensioning the soft tissues. Preferably, to avoid mechanical seal drawbacks, the present example is made with thicknesses that are not too high.

Moreover, the increased thickness of the glenoid components shown in Fig. 8 are defined by a maximum offset provided by respective predetermined offsets 809 between the development plane defined by the first outer edge 803 and the reference plane defined by the second outer edge, that ranges from about: 9%, 18% , 25% with respect to the respective diameter of the glenoid component.

In that respect, the 'normal' thickness of a glenoid component is defined by an offset of about 9% of the diameter of the glenoid component, while the increased thickness of the glenoid components is defined by larger offsets, preferably larger than 15%, more preferably larger than 25%, as also exemplified with reference to Fig. 5 above.

Preferably the longitudinal distance is comprised between 2 and 10 mm, more preferably comprised between 4 and 6 mm.

However, nothing prevents the use of more resistant components with different thicknesses, included in the scope of protection defined by the appended claims.

Figure 10 represents an exploded view of an exemplifying shape of the glenoid part of a reverse shoulder prosthesis comprising a glenoid component 100 comprising a glenoid coupling element 110 and grafted with fixing screws 715, a bone pin 111 and a prosthetic hemisphere 116.

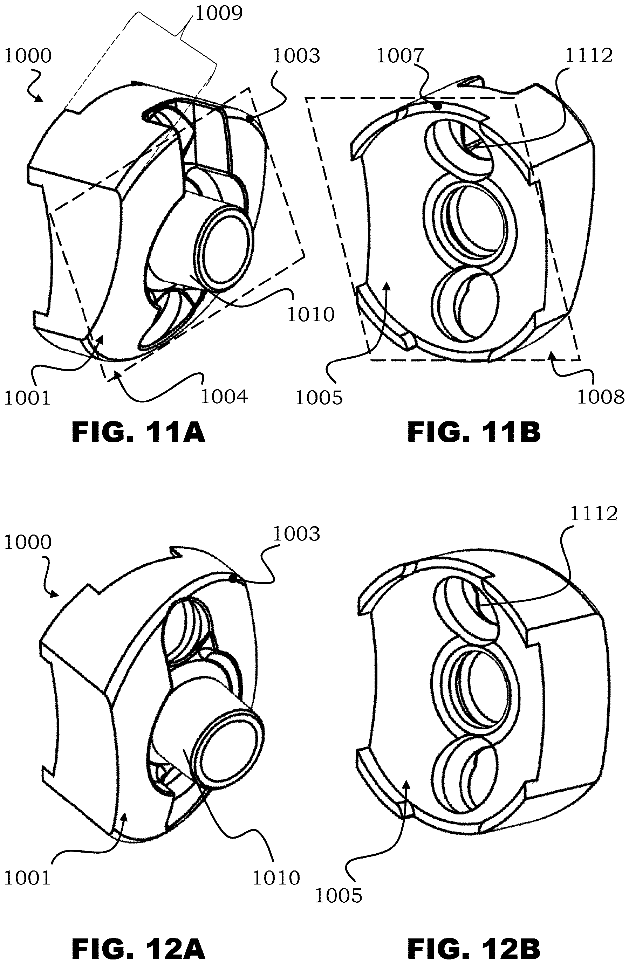

In the event that the surgeon deems it necessary to use an anatomical prosthesis and not a reverse prosthesis, for example in the case of a young patient with an intact rotator cuff, a further example of glenoid component 1000 is provided, which is represented in two different variants in Figures 11A and 11B, and 12A and 12B.

In particular, Figures 11A and 11B represent a configuration for bone deficits in the inferior-superior direction (I-S), whereas Figures 12A and 12B represent a configuration for anterior-posterior bone deficits (A-P).

As it may be seen, this example in both the represented variants provides both the first outer edge 1003 of the glenoid coupling surface 1001 and the second outer edge 1007 of the substantially oval-shaped prosthetic coupling surface 1005. Two variants are required since it is not possible to rotate the glenoid component 1000 due to asymmetry issues analogously to the embodiment with circular outer edges 103 and 107.

As this is an anatomic prosthesis, the glenoid component 1000 must be positioned vertically on the glenoid to anatomically reproduce the surface. In the case of hybrid defects, for instance upper-posterior with a 45° inclination, it is therefore necessary to select one of the two conditions, either posterior or superior, and prepare the seat with a milling operation until the defect is eliminated. Alternatively, however, it is possible to develop a dedicated glenoid component 1000 for a specific bone deficit.

Even in this example the glenoid component 1000 comprises a substantially truncated cone-shaped glenoid coupling element 1010, protruding from the glenoid coupling surface 1001 and being adapted to be coupled to a bone pin 1111, and which develops along an axis that is substantially perpendicular to a un reference plane 1008. Even in this example it is possible to modularly couple a bone pin 1111 of various lengths. It is possible to adopt the same glenoid component both in a configuration of the reverse type, as well as in the case of an anatomical prosthesis, since the geometry of the glenoid coupling element 1000 is similar. This is particularly useful in the event that a conversion is required, namely a transition to an implant of the reverse type after a first implant of the anatomical type.

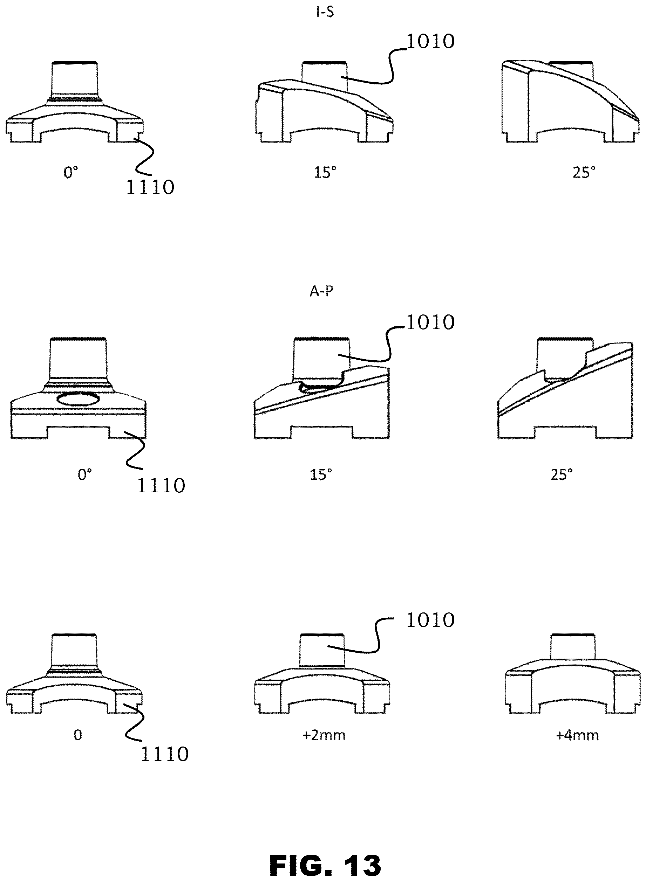

The glenoid component 1000, as visible in Figure 13, provides same features as the previous embodiments and examples. In particular, variants with a predetermined offset 1009 configured to vary a longitudinal distance along the perimeters of a first outer edge 1003 and a second outer edge 1007, defining an angle α between a development plane 1004 and a reference plane 1008, are visible. Even in this case an angle α range comprised between 5° and 25° is preferable, but nothing prevents the adoption of different angles.

Variants with a predetermined offset 1009 configured to maintain the longitudinal distance along the perimeters of the first outer edge 1003 and the second outer edge 1007 substantially constant, in cases of central erosion, are further visible. Even in this case a range of longitudinal distance comprised between 4mm and 6mm is preferable, but nothing prevents from providing different longitudinal distances.

In all cases a constant curvature radius R is provided, with the same characteristics regarding the convexity curvature center described for the corresponding embodiments and examples described above.

Retentive elements 1110 protruding from the prosthetic coupling surface 1001 are preferably further provided.

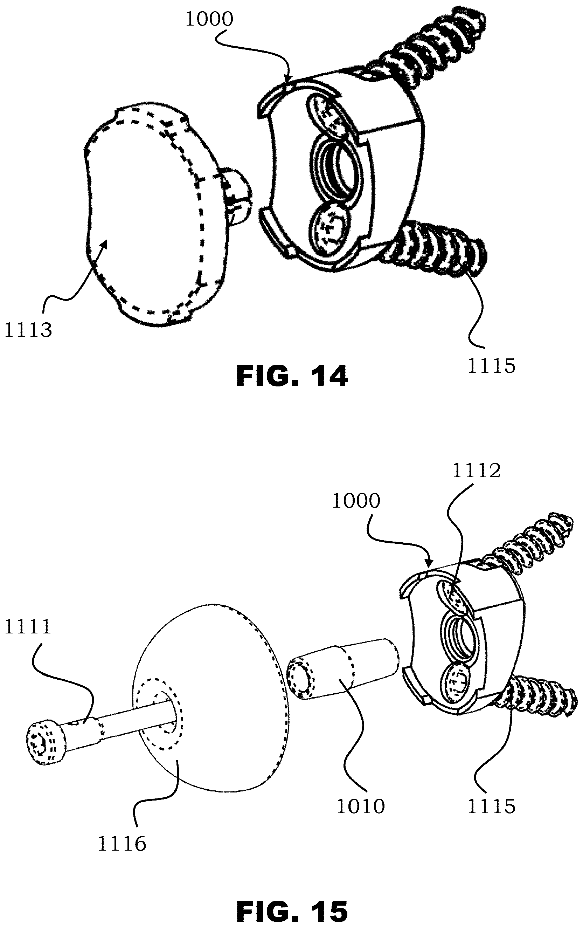

Said retentive elements 1110 are preferably adapted, as visible in Figure 14, to house and retain an anatomic liner 1113, namely a coating will replicate the surface on which the humeral head will articulate. Said element protects and reduces the impact and allows the coupling between humeral head and glenoid. Preferably the anatomic liner 1111 is made of polyethylene. In Figure 14 it is further visible that the present example, given the particular conformation, allows the use of two fixing screws 1115 and thus provides only two holes 1112.

Figure 15 represents an exploded view of an exemplifying shape of the glenoid part of a reverse shoulder prosthesis comprising a glenoid component 1000 comprising a glenoid coupling element 1010 and grafted with fixing screws 1115, a bone pin 1111 and a prosthetic hemisphere 1116.

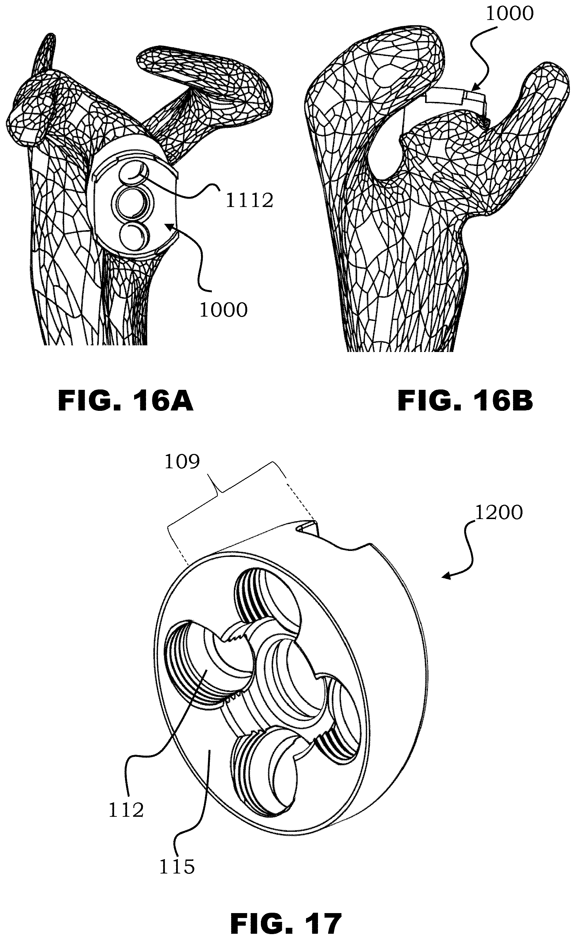

In Figures 16A and 16B a possible exemplifying application of the glenoid component 1000 on a scapula is visible. It is possible to make the glenoid component 1000 of various length and width dimensions in order to replicate the correct geometry of the glenoid for the anatomical implant, and to consequently adopt anatomical liners of different sizes.

In case the surgeon provides for the use of an angular locking screw, an alternative embodiment 1200 is further provided as represented in Figures 17, 18, 19A and 19B, in which the holes 112 are suitable for housing respective angular locking screws 1201.

According to said embodiment, the predetermined offset 109 at the prosthetic coupling surface 105 of the glenoid component 1200 comprises a first offset 109A at the holes 112 and a second offset 109B, at the glenoid coupling element 110. The first offset 109A is preferably comprised between 1mm and 7mm, even more preferably between 2mm and 4mm. In this embodiment, it is observed that the first offset 109A represents an additional thickness, whereas the second offset 109B substantially corresponds to the entire thickness 109 of the other embodiments previously described. The first offset 109A thus acts as an additional thickness and allows introducing a locking element for the angular locking screws 1201.

The portion of the glenoid component 1200 comprising the glenoid coupling element 110 remains unchanged by not introducing any lateralization, whereas the glenoid component 100 provides an offset at the holes 112 that is configured to provide a space for poly-axial locking of the screws 1201.

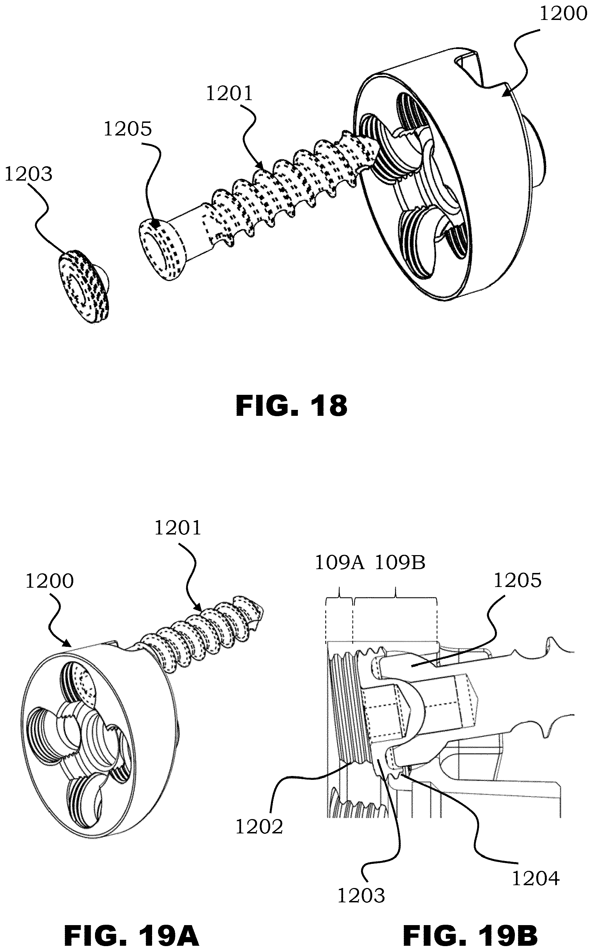

As better visible in the sectional view of Figure 19B, the first offset 109A comprises in this embodiment a first threaded portion 1202, adapted to house a closure element 1203 that is peripherally threaded. The peripherally threaded closure element 1203 is similar to a threaded covering and locking cap for the screw 1201.

The second offset 109B comprises a second threaded portion 1204 adapted to receive a head 1205 of one of the angular locking screws 1201. In the embodiment illustrated, the figures in particular represent a hemispherical head of the screw 1201 with hexagonal recess in which a related peripherally threaded closure element 1203 is inserted, said embodiment being, as stated, exemplifying and non-limiting.

Specifically, the fixing screw 1201 is oriented and screwed in the same way as the previously described embodiments, while the closure element 1203 will allow its angular locking once introduced. The closure element 1203 is thus screwed until it abuts onto the head of the angle locking screw 1201, compressing and locking it polyaxially.

Clearly, the present solution may be applied at all of the holes 112 or at a subset thereof.

In a variant, nothing prevents from providing a second non-threaded offset 109B and only providing a first threaded offset 109A, in particular in the case of not particularly accentuated angles desired for the screw 1201, angles for which the same screw 1201 is mostly protruding.

It is further possible to provide a further variant with a different locking solution, for example by adopting fixing screws with an oversized head comprising an expandable elastic element. In this case, the poly-axial locking of the screw 1201 could be obtained by expanding the elastic element within the first offset 109A, without the need for the latter to provide threaded portions.

The present invention further provides at least one shoulder prosthesis comprising a glenoid component according to one of the embodiments and variants of the invention and at least one related prosthetic component associated with the glenoid component, such as for instance the above-described anatomic liner 1113.

Advantageously, the present invention allows for a range of possibilities in correcting bone deficits with standard and tested elements as suitable for the specific clinical conditions of a patient.

Furthermore, the present invention combines peculiarities under different conditions, with different characteristics for specific bone deficits, and a universality of use in particular by providing a surface adapted to couple on the convex glenoid with constant radius, so as to allow the adoption of a single tool by of a surgeon in the preparatory phase.

Advantageously, the present invention is suitable for different conditions of bone deficits, whether due to angular erosion or due to central erosion.

Advantageously, the present invention allows optimizing time and precision of surgical operations peculiar to the shoulder.

Still advantageously, the present invention provides a relatively simple and intuitive application for a surgeon.

Still advantageously, the present invention provides for coupling with existing devices, such as bone pins, also allowing a coupling with several bone pins of various lengths with a single glenoid component.

Finally, advantageously the present invention does not have particularly complex structural characteristics, making it suitable for large-scale production, with obvious impact also from an economic point of view.

It should also be noted that the different embodiments each further present specific and peculiar advantages as described.

It is evident that, where there are no technical incompatibilities evident to the skilled person, the configurations of specific elements described with reference to certain embodiments can be used in other embodiments described herein.

For instance, it is possible to use a trabecular-porous structure 601 even in the embodiment with an oval peripheral structure.

## Claims

1. Glenoid component (100, 600, 1200) for shoulder prosthesis, comprising:
- a glenoid coupling surface (101), adapted to be fixed to a bone and comprising a first convex portion (102) having a first outer edge (103) which defines a development plane (104);
- a prosthetic coupling surface (105), opposite said glenoid coupling surface (101) and adapted to house a second prosthetic component of said shoulder prosthesis, said prosthetic coupling surface (105) comprising a second portion (106) having a second outer edge (107) which defines a reference plane (108);
wherein said glenoid component (100) provides a predetermined offset (109) between said development plane (104) and said reference plane (108), wherein said predetermined offset (109) is configured to vary a longitudinal distance along the perimeters of said first outer edge (103) and of said second outer edge (107), defining an angle (α) between said development plane (104) and said reference plane (108), said predetermined offset (109) defining an increased thickness of said glenoid component (100, 600, 1200) for compensating a bone deficit,
wherein said first outer edge (103) of said glenoid coupling surface (101) is substantially circular, and wherein said second outer edge (107) of said prosthetic coupling surface (105) is substantially circular,
**characterized in that** said glenoid component (100) comprises a substantially truncated cone-shaped glenoid coupling element (110), projecting from said glenoid coupling surface (101) and being adapted to be coupled to a bone pin (111) of said shoulder prosthesis, said glenoid coupling element (110) developing along an axis which is substantially perpendicular to said reference plane (108),
**and in that** said substantially circular first outer edge (103) and said substantially circular second outer edge (107) are configured so as to allow an orientation of the glenoid component (100, 600, 1200) in a continuous way, covering all types of bone defects over 360°.

2. Glenoid component (100, 600, 1200) according to claim 1, wherein said angle (α) is comprised between 2° and 30°, preferably comprised between 5° and 25°, more preferably available with increases by 5°.

3. Glenoid component (100, 600, 1200) according to claim 1 or 2, wherein said first convex portion (102) provides a substantially constant curvature radius (R), preferably comprised between 25 and 35 mm, more preferably between 27 and 30 mm, and wherein said second portion (106) is preferably concave.

4. Glenoid component (100, 600, 1200) according to any one of claims 1 to 3, further comprising at least two holes (112), preferably four holes (112), adapted to receive fixing screws (715) of said glenoid component of said shoulder prosthesis.

5. Glenoid component (100, 600, 1200) according to claim 4, wherein said holes (112) are adapted to house respective angular locking screws (1201).

6. Glenoid component (100, 600, 1200) according to claim 5, wherein said predetermined offset (109) comprises a first offset (109A) at said holes (112) and a second offset (109B) at a glenoid coupling element (110).

7. Glenoid component (100, 600, 1200) according to claim 6, wherein said first offset (109A) comprises a first threaded portion, adapted to house a peripherally threaded closure element (1203), and preferably wherein said second offset (109B) comprises a second threaded portion, adapted to house a head (1205) of a respective angular locking screw (1201).

8. Glenoid component (100, 600, 1200) according to any one of claims 1 to 7, wherein said glenoid coupling surface (101) comprises a trabecular porous structure (601), adapted to increase the stability and osteo-integration of said glenoid component (100).

9. Glenoid component (100, 600, 1200) according to any one of claims 1 to 8, further comprising retentive elements protruding from said prosthetic coupling surface, preferably said retentive elements being adapted to house and retain an anatomic liner.

10. Glenoid component (100, 600, 1200) according to any one of claims 1 to 9, wherein a set of said glenoid components (100, 600, 1200) provides respective different predetermined offsets (109) so as to define a plurality of different standard increased thicknesses for compensating different bone deficits.

11. A set of glenoid components (100, 600, 1200) according to any one of claims 1 to 10, each glenoid component of said set providing a respective predetermined offset (109), so as to define a plurality of standard increased thicknesses for compensating different bone deficits.

12. Shoulder prosthesis comprising:
- a glenoid component (100, 600, 1200) according to any one of claims 1 to 10;
- at least one second prosthetic component connected to said glenoid component (100, 600, 1200).

## Patentansprüche

1. Glenoidkomponente (100, 600, 1200) für eine Schulterprothese, aufweisend:
- eine Glenoid-Kopplungsfläche (101), die zur Befestigung an einem Knochen ausgebildet ist und einen ersten konvexen Bereich (102) mit einem ersten Außenrand (103) aufweist, der eine Abwicklungsebene (104) bildet;
- eine Prothesen-Kopplungsfläche (105), die der Glenoid-Kopplungsfläche (101) gegenüberliegt und dazu ausgebildet ist, eine zweite Prothesenkomponente der Schulterprothese aufzunehmen, wobei die Prothesen-Kopplungsfläche (105) einen zweiten Bereich (106) mit einem zweiten Außenrand (107) aufweist, der eine Referenzebene (108) bildet;
wobei die Glenoidkomponente (100) einen vorbestimmten Versatz (109) zwischen der Abwicklungsebene (104) und der Referenzebene (108) bereitstellt, wobei der vorbestimme Versatz (109) dazu ausgebildet ist, einen Längsrichtungsabstand entlang der Umfänge des ersten Außenrands (103) und des zweiten Außenrands (107) zu variieren, um einen Winkel (α) zwischen der Abwicklungsebene (104) und der Referenzebene (108) zu bilden, wobei der vorbestimme Versatz (109) eine erhöhte Dicke der Glenoidkomponente (100, 600, 1200) bildet, um ein Knochendefizit auszugleichen,
wobei der erste Außenrand (103) der Glenoid-Kopplungsfläche (101) im Wesentlichen kreisförmig ist und wobei der zweite Außenrand (107) der Prothesen-Kopplungsfläche (105) im Wesentlichen kreisförmig ist,
**dadurch gekennzeichnet, dass** die Glenoidkomponente (100) ein im Wesentlichen kegelstumpfförmiges Glenoid-Kopplungselement (110) aufweist, das von der Glenoid-Kopplungsfläche (101) hervorsteht und dazu ausgebildet ist, mit einem Knochenstift (111) der Schulterprothese gekoppelt zu werden, wobei das Glenoid-Kopplungselement (110) entlang einer Achse abgewickelt wird, die im Wesentlichen rechtwinklig zu der Referenzebene (108) ist,
und dass der im Wesentlichen kreisförmige erste Außenrand (103) und der im Wesentlichen kreisförmige zweite Außenrand (107) derart ausgebildet sind, dass sie eine Orientierung der Glenoidkomponente (100, 600, 1200) in kontinuierlicher Weise zulassen, so dass alle Arten von Knochendefekten über 360° abgedeckt werden.

2. Glenoidkomponente (100, 600, 1200) nach Anspruch 1,
wobei der Winkel (a) zwischen 2° und 30° liegt, vorzugsweise zwischen 5° und 25° liegt, stärker bevorzugt mit Steigerungen um 5° verfügbar ist.

3. Glenoidkomponente (100, 600, 1200) nach Anspruch 1 oder 2,
wobei der erste konvexe Bereich (102) einen im Wesentlichen konstanten Krümmungsradius (R) bereitstellt, der vorzugsweise zwischen 25 und 35 mm liegt und stärker bevorzugt zwischen 27 und 30 mm liegt, und wobei der zweite Bereich (106) vorzugsweise konkav ist.

4. Glenoidkomponente (100, 600, 1200) nach einem der Ansprüche 1 bis 3,
die ferner mindestens zwei Löcher (112), vorzugsweise vier Löcher (112) aufweist, die zum Aufnehmen von Befestigungsschrauben (715) der Glenoidkomponente der Schulterprothese ausgebildet sind.

5. Glenoidkomponente (100, 600, 1200) nach Anspruch 4,
wobei die Löcher (112) dazu ausgebildet sind, jeweilige Winkelverriegelungsschrauben (1201) aufzunehmen.

6. Glenoidkomponente (100, 600, 1200) nach Anspruch 5,
wobei der vorbestimmte Versatz (109) einen ersten Versatz (109A) bei den Löchern (112) und einen zweiten Versatz (109B) bei einem Glenoid-Kopplungselement (110) umfasst.

7. Glenoidkomponente (100, 600, 1200) nach Anspruch 6,
wobei der erste Versatz (109A) einen ersten Gewindebereich aufweist, der dazu ausgebildet ist, ein peripher mit Gewinde versehenes Verschlusselement (1203) aufzunehmen, und wobei vorzugsweise der zweite Versatz (109B) einen zweiten Gewindebereich aufweist, der dazu ausgebildet ist, einen Kopf (1205) einer jeweiligen Winkelverriegelungsschraube (1201) aufzunehmen.

8. Glenoidkomponente (100, 600, 1200) nach einem der Ansprüche 1 bis 7,
wobei die Glenoid-Kopplungsfläche (101) eine trabekuläre poröse Struktur (601) besitzt, die dazu ausgebildet ist, die Stabilität und Osteointegration der Glenoidkomponente (100) zu steigern.

9. Glenoidkomponente (100, 600, 1200) nach einem der Ansprüche 1 bis 8,
die ferner Halteelemente aufweist, die von der Prothesen-Kopplungsfläche hervorstehen, wobei die Halteelemente (1110) vorzugsweise zum Aufnehmen und Festhalten eines anatomischer Liners ausgebildet sind.

10. Glenoidkomponente (100, 600, 1200) nach einem der Ansprüche 1 bis 9, wobei ein Satz der Glenoidkomponenten (100, 600, 1200) jeweils unterschiedliche vorbestimmte Versätze (109) bereitstellt, um eine Mehrzahl unterschiedlicher erhöhter Standarddicken zum Kompensieren von unterschiedlichen Knochendefiziten zu bilden.

11. Satz von Glenoidkomponenten (100, 600, 1200) nach einem der Ansprüche 1 bis 10, wobei jede Glenoidkomponente des Satzes einen jeweiligen vorbestimmten Versatz (109) bereitstellt, um eine Mehrzahl von erhöhten Standarddicken zum Kompensieren von unterschiedlichen Knochendefiziten zu bilden.

12. Schulterprothese, aufweisend:
- eine Glenoidkomponente (100, 600, 1200) nach einem der Ansprüche 1 bis 10;
- mindestens eine zweite Prothesenkomponente, die mit der Glenoidkomponente (100, 600, 1200) verbunden ist.

## Revendications

1. Composant glénoïde (100, 600, 1200) pour une prothèse d'épaule, comprenant :
_une surface de couplage glénoïde (101), adaptée pour être fixée à un os et comprenant une première portion convexe (102) ayant un premier bord extérieur (103) qui définit un plan de développement (104) ;
_une surface de couplage prothétique (105), opposée à ladite surface de couplage glénoïde (101) et adaptée pour loger un second composant prothétique de ladite prothèse d'épaule, ladite surface de couplage prothétique (105) comprenant une seconde portion (106) ayant un second bord extérieur (107) qui définit un plan de référence (108) ;
dans lequel ledit composant glénoïde (100) fournit un décalage prédéterminé (109) entre ledit plan de développement (104) et ledit plan de référence (108), dans lequel ledit décalage prédéterminé (109) est configuré pour faire varier une distance longitudinale le long des périmètres dudit premier bord extérieur (103) et dudit second bord extérieur (107), définissant un angle (a) entre ledit plan de développement (104) et ledit plan de référence (108), ledit décalage prédéterminé (109) définissant une épaisseur accrue dudit composant glénoïde (100, 600, 1200) pour compenser un déficit osseux,
dans lequel ledit premier bord extérieur (103) de ladite surface de couplage glénoïde (101) est sensiblement circulaire, et dans lequel ledit second bord extérieur (107) de ladite surface de couplage prothétique (105) est sensiblement circulaire,
**caractérisé en ce que** ledit composant glénoïde (100) comprend un élément de couplage glénoïde (110) sensiblement en forme de cône tronqué, faisant saillie de ladite surface de couplage glénoïde (101) et étant adapté pour être couplé à une broche pour os (111) de ladite prothèse d'épaule, ledit élément de couplage glénoïde (110) se développant selon un axe qui est sensiblement perpendiculaire audit plan de référence (108),
et **en ce que** ledit premier bord extérieur (103) sensiblement circulaire et ledit second bord extérieur (107) sensiblement circulaire sont configurés de manière à permettre une orientation du composant glénoïde (100, 600, 1200) de manière continue, couvrant tous les types de défauts osseux supérieurs à 360°.

2. Composant glénoïde (100, 600, 1200) selon la revendication 1, dans lequel ledit angle (a) est compris entre 2° et 30°, préférentiellement compris entre 5°et 25°, plus préférentiellement disponible avec des augmentations de 5°.

3. Composant glénoïde (100, 600, 1200) selon la revendication 1 ou 2, dans lequel ladite première portion convexe (102) fournit un rayon de courbure (R) sensiblement constant, préférentiellement compris entre 25 et 35 mm, plus préférentiellement entre 27 et 30 mm, et dans lequel ladite seconde portion (106) est préférentiellement concave.

4. composant glénoïde (100, 600, 1200) selon l'une quelconque des revendications 1 à 3, comprenant en outre au moins deux trous (112), préférentiellement quatre trous (112), adaptés pour recevoir des vis de fixation (715) dudit composant glénoïde de ladite prothèse d'épaule.

5. Composant glénoïde (100, 600, 1200) selon la revendication 4, dans lequel lesdits trous (112) sont adaptés pour loger des vis de verrouillage angulaires (1201) respectives.

6. Composant glénoïde (100, 600, 1200) selon la revendication 5, dans lequel ledit décalage prédéterminé (109) comprend un premier décalage (109A) au niveau desdits trous (112) et un second décalage (109B) au niveau d'un élément de couplage glénoïde (110).

7. Composant glénoïde (100, 600, 1200) selon la revendication 6, dans lequel ledit premier décalage (109A) comprend une première portion filetée, adaptée pour loger un élément de fermeture fileté périphérique (1203), et préférentiellement dans lequel ledit second décalage (109B) comprend une seconde portion filetée, adaptée pour loger une tête (1205) d'une vis de verrouillage angulaire (1201) respective.

8. Composant glénoïde (100, 600, 1200) selon l'une quelconque des revendications 1 à 7, dans lequel ladite surface de couplage glénoïde (101) comprend une structure poreuse trabéculaire (601), adaptée pour augmenter la stabilité et l'ostéo-intégration dudit composant glénoïde (100).

9. Composant glénoïde (100, 600, 1200) selon l'une quelconque des revendications 1 à 8, comprenant en outre des éléments rétentifs faisant saillie de ladite surface de couplage prothétique, préférentiellement lesdits éléments rétentifs étant adaptés pour loger et retenir un manchon anatomique.

10. Composant glénoïde (100, 600, 1200) selon l'une quelconque des revendications 1 à 9, dans lequel un ensemble desdits composants glénoïdes (100, 600, 1200) fournit des décalages prédéterminés (109) différents respectifs de manière à définir une pluralité d'épaisseurs accrues types différentes pour compenser des déficits osseux différents.

11. Ensemble de composants glénoïdes (100, 600, 1200) selon l'une quelconque des revendications 1 à 10, chaque composant glénoïde dudit ensemble fournissant un décalage prédéterminé (109) respectif, de manière à définir une pluralité d'épaisseurs accrues types pour compenser des déficits osseux différents.

12. Prothèse d'épaule comprenant :
_ un composant glénoïde (100, 600, 1200) selon l'une quelconque des revendications 1 à 10 ;
_ au moins un second composant prothétique relié audit composant glénoïde (100, 600, 1200).
